# EUROPÄISCHE PATENTSCHRIFT

(11) **EP 0 609 242 B1**
(45) Veröffentlichungstag und Bekanntmachung des Hinweises auf die Patenterteilung: **18.12.1996**
(21) Anmeldenummer: 92919916.4
(22) Anmeldetag: 21.09.1992
(51) Int. Cl.: C12N 15/15, A61K 38/55, C12P 21/02

(54) **NEUES THROMBININHIBITORISCHES PROTEIN AUS ZECKEN**
NEW THROMBIN-INHIBITING PROTEIN ISOLATED FROM TICKS
NOUVELLE PROTEINE INHIBITRICE DE LA THROMBINE PROVENANT DE TIQUES

(30) Priorität: 22.10.1991 DE 4134814
(43) Veröffentlichungstag der Anmeldung: 10.08.1994
(73) Patentinhaber: BASF Aktiengesellschaft, 67063 Ludwigshafen (DE)
(72) Erfinder: FRIEDRICH, Thomas, D-6100 Darmstadt (DE); BIALOJAN, Siegfried, D-6836 Oftersheim (DE); BOLLSCHWEILER, Claus, D-6900 Heidelberg (DE); KUENAST, Christoph, D-6701 Otterstadt (DE)
(86) Internationale Anmeldenummer: EP9202179
(87) Internationale Veröffentlichungsnummer: WO9308282

(56) Entgegenhaltungen:
- WO-A-91/04275
- JOURNAL OF BIOLOGICAL CHEMISTRY (MICROFILMS), Band 265, Nr. 29, 15. Oktober 1990, Baltimore, MD (US); M. NEEPER et al., Seiten 17746-17752

### SEQUENCE LISTING

(1) GENERAL INFORMATION:
   (i) APPLICANT:
      (A) NAME: BASF Aktiengesellschaft
      (B) STREET: Carl-Bosch-Strasse 38
      (C) CITY: Ludwigshafen
      (E) COUNTRY: Bundesrepublik Deutschland
      (F) POSTAL CODE (ZIP): D-6700
      (G) TELEPHONE: 0621/6048526
      (H) TELEFAX: 0621/6043123
      (I) TELEX: 1762175170
   (ii) TITLE OF INVENTION: Neues thrombininhibitorisches Protein aus Zecken
   (iii) NUMBER OF SEQUENCES: 7
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER:
(2) INFORMATION FOR SEQ ID NO:1:
   (i) SEQUENCE CHARACTERISTICS:
      (A) LENGTH: 33 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (v) FRAGMENT TYPE: N-terminal
   (vi) ORIGINAL SOURCE:
      (A) ORGANISM: Ornithodoros moubata
      (D) DEVELOPMENTAL STAGE: adult
      (F) TISSUE TYPE: whole animals
   (ix) FEATURE:
      (A) NAME/KEY: Peptide
      (B) LOCATION: 1..33
   (xi) SEQUENCE DESCRIPTION: SEQ ID NO:1:
   Leu Asn Val Leu Cys Asn Asn Pro His Thr Ala Asp Cys Asn Asn Asp
   1               5                   10                  15
   Ala Gln Val Asp Arg Tyr Phe Arg Glu Gly Thr Thr Cys Leu Met Ser
               20                  25                  30
   Pro
(2) INFORMATION FOR SEQ ID NO:2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: YES
   (iv) ANTI-SENSE: NO
   (xi) SEQUENCE DESCRIPTION: SEQ ID NO:2:
   TGYAAYAAYC CNCAYACNGC                                    20
(2) INFORMATION FOR SEQ ID NO:3:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: YES
   (iv) ANTI-SENSE: NO
   (xi) SEQUENCE DESCRIPTION: SEQ ID NO:3:
   CCNCAYACNG CNGAYTGYAA 20
(2) INFORMATION FOR SEQ ID NO:4:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:4:
   TTGAATGTGT TGTCCAATAA TCCGCATACG GCCGATTGCA AC 42
(2) INFORMATION FOR SEQ ID NO:5:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO:5:
   GGGGGGGTCG ACGTGCTAGT ACCTTACATG TTT 33
(2) INFORMATION FOR SEQ ID NO:6:
   (i) SEQUENCE CHARACTERISTICS:
      (A) LENGTH: 291 base pairs
      (B) TYPE: nucleic acid
      (C) STRANDEDNESS: single
      (D) TOPOLOGY: linear
   (ii) MOLECULE TYPE: cDNA to mRNA
   (iii) HYPOTHETICAL: NO
   (iv) ANTI-SENSE: NO
   (vi) ORIGINAL SOURCE:
      (A) ORGANISM: Ornithodoros moubata
      (D) DEVELOPMENTAL STAGE: adult
      (F) TISSUE TYPE: whole animals
   (ix) FEATURE:
      (A) NAME/KEY: CDS
      (B) LOCATION: 1..277
   (xi) SEQUENCE DESCRIPTION: SEQ ID NO:6:
   TTG AAT GTG TTG TCC AAT AAT CCG CAT ACG GCC GAT TGC AAC AAT GAT        48
   Leu Asn Val Leu Ser Asn Asn Pro His Thr Ala Asp Cys Asn Asn Asp
     1               5                  10                  15
   GCA CAG GTT GAC AGA TAT TTT AGG GAG GGG ACA ACG TGC CTA ATG TCC        96
   Ala Gln Val Asp Arg Tyr Phe Arg Glu Gly Thr Thr Cys Leu Met Ser
                20                  25                  30
   CCA GCA TGC ACG AGC GAA GGA TAC GCC TCT CAG CAC GAA TGT CTC AGG       144
   Pro Ala Cys Thr Ser Glu Gly Tyr Ala Ser Gln His Glu Cys Leu Arg
            35                  40                  45
   CCT GCT TTG TTG GCG GGG AAG ACC ACA GCA GTG AAA TGC ACA GCT CAT       192
   Pro Ala Leu Leu Ala Gly Lys Thr Thr Ala Val Lys Cys Thr Ala His
        50                  55                  60
   GCC TTG GTG ACC CGC CCA CTT CCT GCG CGG AAG GCA CGG ACA TCA CCT       240
   Ala Leu Val Thr Arg Pro Leu Pro Ala Arg Lys Ala Arg Thr Ser Pro
    65                  70                  75                  80
   ACT ACG ATT CTG ATA GCA AAA CAT GTA AGG TAC TAG C AGGTCGACCC          287
   Thr Thr Ile Leu Ile Ala Lys His Val Arg Tyr  *
                        85                  90
   CCCC                                                                  291
(2) INFORMATION FOR SEQ ID NO:7:
   (i) SEQUENCE CHARACTERISTICS:
      (A) LENGTH: 92 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Leu Asn Val Leu Ser Asn Asn Pro His Thr Ala Asp Cys Asn Asn Asp
    1               5                  10                  15
   Ala Gln Val Asp Arg Tyr Phe Arg Glu Gly Thr Thr Cys Leu Met Ser
               20                  25                  30
   Pro Ala Cys Thr Ser Glu Gly Tyr Ala Ser Gln His Glu Cys Leu Arg
           35                  40                  45
   Pro Ala Leu Leu Ala Gly Lys Thr Thr Ala Val Lys Cys Thr Ala His
       50                  55                  60
   Ala Leu Val Thr Arg Pro Leu Pro Ala Arg Lys Ala Arg Thr Ser Pro
   65                  70                  75                  80
   Thr Thr Ile Leu Ile Ala Lys His Val Arg Tyr  *
                   85                  90

## Beschreibung

Die vorliegende Erfindung betrifft ein neues thrombininhibitorisches Protein aus Zecken sowie Verfahren zu dessen Herstellung.

Thrombin-Inhibitoren sind wichtige therapeutische Substanzen, die beispielsweise zur Prophylaxe oder Behandlung von Thrombosen oder arteriellen Reokklusionen verwendet werden.

In der deutschen Offenlegungsschrift DE 39 31 839 wird ein Thrombinininhibitor, der aus der Lederzecke Ornithodoros moubata isoliert worden ist, beschrieben. Dieses Protein besitzt ein Molekulargewicht von etwa 15000 Dalton, einen isoelektrischen Punkt von pH 4-5 und die N-terminale Aminosäuresequenz SDYEFPPPKKXRPG.

In der europäischen Offenlegungsschrift EP 345 614 wird der thrombininhibitorische Wirkstoff Amblyommin beschrieben, der aus Schildzecken isoliert wird. Es handelt sich dabei um ein Protein mit einem Molekulargewicht von 20 000 - 30 000 Dalton und einem isoelektrischen Punkt zwischen 5,05 und 5,65.

Bisher wurde jedoch noch kein Protein mit thromübininhibitorischer Wirkung gefunden, das als Arzneimittel hinsichtlich hoher Wirksamkeit, fehlender Antigenizität, langer biologischer Halbwertzeit, geringer Nebenwirkung wie z.B. Blutungsneigung, vorteilhaft geeignet ist.

Der Erfindung lag daher die Aufgabe zugrunde, neue Thrombin-Inhibitoren zur Verfügung zu stellen, die als Arzneimittel hinsichtlich der oben genannten Eigenschaften geeignet sind.

Demgemäß wurde ein neues thrombininhibitorisches Protein aus Zecken isoliert.

Das neue Protein besitzt folgende physikochemischen Eigenschaften. Durch Molekularsiebchromatographie wird ihm ein Molekulargewicht von 22000 - 28000 Dalton zugeordnet. In einem SDS-Polyacrylamidgel wird ein Molekulargewicht von 8000 ± 1500 Dalton bestimmt. Die Bestimmung des isoelektrischen Punktes ergibt einen pH-Wert von kleiner als 3,8.

Das Protein bindet spezifisch an eine Thrombin-Affinitätssäule. Es hemmt die biologische Aktivität von Thrombin in einem in-vitro Enzymtest.

In einem Polyacrylamidgel läßt sich die Proteinbande durch Silberfärbung nicht anfärben; sie ist nur als nicht-gefärbter Fleck auf einem gefärbten Hintergrund sichtbar.

Folgende N-terminale Aminosäuresequenz wurde von dem Protein bestimmt (SEQ ID NO:1):

Leu-Asn-Val-Leu-Cys-Asn-Asn-Pro-His-Thr-Ala-Asp-Cys-Asn-Asn-Asp-Ala-Gln-Val-Asp-Arg-Tyr-Phe-Arg-Glu-Gly-Thr-Thr-Cys-Leu-Met-Ser-Pro.

Die gesamte Aminosäuresequenz des neuen Proteins ist in SEQ ID NO: 7 dargestellt.

Weitere erfindungsgemäße Proteine sind solche, die sich von der in SEQ ID NO: 7 dargestellten Aminosäuresequenz durch Austausch von bis zu 10 Aminosäuren oder durch Deletion einzelner Aminosäuren unterscheiden.

Stiche Aminosäuresequenzen lassen sich beispielsweise über ein entsprechend mutiertes Gen leicht gentechnisch herstellen.

Das neue Protein läßt sich aus Zecken der Gattung Ornithodoros isolieren. Hierzu werden die Zecken zweckmäßigerweise in einem Puffer bei pH 6 bis 9, vorzugsweise pH 7 bis 8 aufgenommen und mit einem Homogenisator, vorzugsweise einem Mixer, homogenisiert. Anschließend werden die unlöslichen Bestandteile abgetrennt, bevorzugt abzentrifugiert.

Aus der so erhaltenen Lösung kann das Protein weiter gereinigt werden, indem mit einem Fällungsmittel, bevorzugt Trichloressigsäure, andere Proteine aus der Lösung ausgefällt und anschließend abgetrennt werden.

Die weitere Reinigung des Proteins kann über chromatographische Methoden, bevorzugt Ionenaustauschchromatographie und/ oder Affinitätschromatographie erfolgen. Besonders bevorzugt ist ein Reinigungsschritt über Thrombin-Affinitätschromatographie.

Die Reinigung des Proteins kann über einen Thrombin-Aktivitätstest verfolgt werden. Hierzu benutzt man zweckmäßigerweise einen optischen Test, bei dem ein chromogenes Substrat, beispielsweise Chromozym T, durch Thrombin umgesetzt wird. Die das neue Protein enthaltenden Fraktionen können bei Zugabe in diesen optischen Test an ihrer Thrombin inhibierenden Wirkung erkannt werden.

Besonders geeignet zur Herstellung des erfindungsgemäßen Proteins sind gentechnische Verfahren.

Hierzu wird in an sich bekannter Weise eine cDNA-Genbank aus der Zecke angelegt. Aus dieser Genbank kann das für das erfindungsgemäße Protein kodierende Gen isoliert werden, indem man beispielsweise eine DNA-Probe herstellt, deren Sequenz von der oben beschriebenen N-terminalen Aminosäuresequenz durch Rückübersetzung gemäß dem genetischen Code erhalten wird. Durch Hybridisierung mit dieser DNA-Probe läßt sich das entsprechende Gen auffinden und isolieren.

Für die Herstellung des entsprechenden Gens kann aber auch die Polymerase-Chain-Reaction (PCR)-Technik eingesetzt werden. Beispielsweise läßt sich mit Hilfe eines Primers, dessen Sequenz durch Rückübersetzung aus der oben beschriebenen N-terminalen Aminosäuresequenz erhalten wurde, und eines zweiten Primers, dessen Sequenz komplementär zum 3'-Ende des cDNA-Genfragments ist, bevorzugt mit der Sequenz Poly(dT), das cDNA-Genfragment für das erfindungsgemäße Protein durch PCR-Technik herstellen. Das entsprechende Gen läßt sich auch isolieren, indem man eine Expressionsgenbank von Zecken anlegt und diese mit einem Antikörper der gegen das erfindungsgemäße Protein gerichtet ist, absucht.

Das Gen für das eingangs beschriebene Protein hat die in SEQ ID NO: 6 dargestellte Nukleotidsequenz.

Außer diesem Gen sind auch solche Nukleotidsequenzen geeignet, die für die in SEQ ID NO: 7 dargestellte Aminosäuresequenz codieren, infolge der Degeneration des gentechnischen Codes jedoch eine andere Nukleotidsequenz als SEQ ID NO: 6 aufweisen. Solche Gene lassen sich durch cehmische Totalsynthese oder durch Mutagenese der SEQ ID NO: 6-Nukleotidsequenz herstellen.

Nachdem das entsprechende Gen isoliert worden ist, kann es durch gentechnische Verfahren in Organismen, z.B. in Bakterien, Hefen, eukaryontischen Zellen, mit Hilfe eines Expressionsvektors in an sich bekannter Weise exprimiert werden. Bevorzugt wird in Prokaryonten, wie z.B. E.coli und mit Hilfe stark exprimierender Vektoren, z.B. unter der Kontrolle des induzierbaren tac-Promotors wie z.B, in Plasmid pMAL-p2 (Protein Fusion und Purification System, GeneExpreß; New England Biolabs) vorhanden, gearbeitet. Dabei kommt es zur periplasmatischen Expression eines Fusionsproteins aus dem Maltose-Bindungsprotein und dem beschriebenen Thrombininhibitor. Der Fusionspartner kann nach Reinigung enzymatisch entfernt werden. Aus diesen rekombinanten Wirtssystemen läßt sich das Protein aufgrund der oben beschriebenen physikochemischen Eigenschaften isolieren.

Die generelle Vorgehensweise zur gentechnischen Herstellung eines neuen Proteins bei bekannter Aminosäurepartialsequenz ist in Lehrbüchern der Gentechnologie, beispielsweise E.L. Winnacker, Gene und Klone, Verlag Chemie, Weinheim, 1984, beschrieben. Die experimentellen Bedingungen für die einzelnen Verfahren wie beispielsweise Anlegen einer Genbank, Hybridisierung, Expression eines Gens sind bei J. Sambrook et al., "Molecular Cloning", Cold Spring Harbor Laboratory, 1989, beschrieben.

Das erfindungsgemäße Protein wird bevorzugt in Form seiner pharmazeutisch annehmbaren Salze verwendet.

Das neue Protein besitzt blutgerinnungshemmende Eigenschaften. Es kann beispielsweise zur Prophylaxe von Thrombosen oder arteriellen Reokklusionen, zur Behandlung von Thrombosen, zur Konservierung von Blut oder bei der extrakorporalen Zirkulation verwendet werden.

Die Erfindung wird durch die folgenden Beispiele weiter veranschaulicht.

### Beispiel 1

### Reinigung des thrombininhibitorischen Proteins aus Zecken

Eine Laborkultur der Zecken (Onithodoros moubata) wurde bei 28°C und 80 % relativer Luftfeuchtigkeit gehalten. In 14-tägigen Abständen wurden die Zecken dadurch gefüttert, daß sie an Kaninchen saugen konnten. Zecken aller Entwicklungsstadien wurden bei -20°C eingefroren.

60 g Zecken wurden mit 400 ml 20 mM Natriumphosphatpuffer, 150 mM NaCl (pH 7,5) homogenisiert. Das Homogenat wurde 15 Minuten bei 7000 U/min (Sorvall RC-5B, Rotor GS-3) zentrifugiert. Der Niederschlag wurde verworfen und der Überstand mit 25 ml 50 Gew.% Trichloressigsäure durch Zutropfen über einen Zeitraum von 15 Minuten versetzt.

Danach wurde zentrifugiert (20 Minuten, 7000 U/min) und der Überstand mit Natronlauge neutralisiert.

Der neutralisierte Überstand wurde in Dialyseschläuche (Ausschlußvolumen 300 Da) gefüllt und mehrfach gegen das 10-fache Volumen 20 mM Natriumphosphat, 150 mM NaCl, pH 8,0 dialysiert.

Die dialysierte Proteinlösung wurde auf eine Q-Sepharose Säule® (Pharmacia) aufgetragen (60 ml/h), die in 20 mM Natriumphosphatpuffer pH 8,0 äquilibriert worden war (Durchmesser 1,5 cm, Höhe 6 cm, Volumen 11 ml).

Es wurde mit 10 Säulenvolumen Äquilibrierungspuffer gewaschen.

Danach wurde ein linearer Gradient von 50 ml 20 mM Natriumphosphat (pH 8,0), nach 50 ml 20 mM Natriumphosphat (pH 8,0), 1 M NaCl angelegt.

Aktive Fraktionen (gemessen durch Thrombininhibition) wurden gesammelt.

Die vereinigten aktiven Fraktionen aus der Q-Sepharose®-Chromatographie wurden auf eine Kupferchelat-Chromatographiesäule (Durchmesser 2,5 cm, Höhe 6 cm, Volumen 30 ml) gegeben, die mit 20 mM Natriumphosphat (pH 8,0), 150 mM NaCl äquilibriert worden war. Die Säule wurde mit 10 Säulenvolumen Äquilibrierungspuffer gewaschen (1,5 ml/min) und mit einem Gradienten von 150 ml 20 mM Natriumphosphat (pH 8,0), 150 mM NaCl nach 150 ml 20 mM Natriumphosphat (pH 8,0), 150 mM NaCl, 100 mM Imidazol eluiert.

Aktive Fraktionen (gemessen durch Thrombininhibition) wurden gesammelt.

Die vereinigten aktiven Fraktionen wurden auf eine Affinitätssäule mit immobilisiertem Thrombin aufgetragen (Durchmesser 1,5 cm, Höhe 6,5 cm, 11,5 ml Säulenvolumen, 60 ml/h). Die Säule wurde gemäß Beispiel 3 hergestellt.

Die Säule wurde mit 20 mM Natriumphosphat pH 7,5 äquilibriert. Nach Auftragen der Proteinlösung wurde die Säule mit 10 Säulenvolumen Äquilibrierungspuffer gewaschen, bis die Absorption bei 280 nm auf Null zurückging.

Danach wurde mit 0,5 M NaCl, 20 mM Natriumphosphatpuffer pH 7,5 gewaschen. Unspezifisch adsorbiertes Material wurde so entfernt.

Spezifisch an Thrombin gebundenes Protein wurde mit 0,1 M Glycin, 0,5 M NaCl pH 2,8 eluiert. Die Säule wurde anschließend sofort wieder mittels Phosphatpuffer auf pH 7,5 eingestellt.

Die einzelnen Fraktionen wurden mit 0,1 M NaOH neutralisiert und auf ihre inhibitorische Wirkung gegen Thrombin untersucht.

Die durch Glycin/NaCl-Puffer pH 2,8 eluierten Fraktionen besaßen thrombininhibierende Wirkung.

Die gesammelten aktiven Fraktionen wurden, nachdem sie neutralisiert worden waren, mit Wasser verdünnt (1:10) und auf eine Mono-Q-Säule® gegeben (Pharmacia, 1 ml Säulenvolumen).

Die Säule wurde äquilibriert mit 20 mM Natriumphosphatpuffer pH 7,5, 150 mM NaCl (Puffer A). Es wurde mit Puffer A gewaschen bis die Absorption auf Null zurückging (10 Minuten). Dann wurde innerhalb von 50 Minuten auf 20 mM Natriumphosphat, pH 7,5, 800 mM NaCl (Puffer B) gewechselt (Fluß 0,5 ml/min).

Thrombin hemmende Fraktionen wurden gesammelt.

Die gesammelten Fraktionen wurden an einer RP 318® (Biorad) HPLC-Chromatographiesäule weiter gereinigt. Die Säule wurde äquilibriert mit 0,1 Gew.% Trifluoressigsäure (TFA) in dest. Wasser. Die vereinigten aktiven Fraktionen wurden auf die Säule aufgetragen. Mit einem Gradienten nach 0,1 % TFA, 100 % Acetonitril während einer Stunde und einer Flußrate von 1 ml/min wurde die Säule eluiert. Die Absorption wurde bei 280 nm bestimmt. Es wurden 0,5 ml Fraktionen gesammelt. Die gesammelten Fraktionen wurden zur Trockene einkonzentriert und in Phosphat gepufferter Salzlösung (PBS) (0,8 g/l NaCl; 0,2 g/l HCl; 0,144 g/l Natriumphosphat; 0,2 g/l Kaliumphosphat, pH 7,5) aufgenommen sowie die inhibitorische Aktivität bestimmt.

Die Proteinbestimmung erfolgte nach der Vorschrift von Bradford (Anal. Biochem., 72, 248-254 (1976)). Als Standardprotein diente Rinderserumalbumin (Boehringer Mannheim).

### Beispiel 2

### Bestimmung der Hemmung von Thrombin durch den Inhibitor

Thrombin (Boehringer Mannheim) wurde zu einer Endkonzentration von 25 mU/ml in Phosphat gepufferter Salzlösung (PBS) (0,8 g/l NaCl; 0,2 g/l HCl; 0,144 g/l Natriumphosphat; 0,2 g/l Kaliumphosphat, pH 7,5) gelöst.

Chromozym TH (Boehringer Mannheim) wurde in 20 ml H₂O/Flasche gelöst.

50 µl Thrombinlösung und 100 µl Chromozym sowie 25 µl Probe oder Puffer wurden in die Näpfe einer Mikrotiterplatte gegeben. Sofort danach wurde zur Zeit 0 und nach 30 Minuten bei 37°C die Absorption bei 405 nm gemessen.

Bei starker Eigenfarbe der Probe wurde eine weitere Kontrolle ohne Thrombin wie oben behandelt.

Durch die Aktivität des Thrombins wird aus dem chromogenen Farbsubstrat ein bei 405 nm absorbierender Farbstoff freigesetzt. Die Hemmung des Thrombins durch einen Thrombininhibitor ist an einer geringeren Absorptionszunahme bei 405 nm erkennbar und wurde mit Hilfe einer Eichkurve quantifieziert.

### Beispiel 3

### Herstellung einer Affinitätssäule mit Thrombin als Ligand

a) Kopplung:
   2 g CNBr aktivierte Sepharose (Pharmacia) wurden mit 200 ml 1 mM HCl auf einer Nutsche gewaschen. Das Gel wurde in 100 mM NaHCO₃, 500 mM NaCl pH 8,3 aufgenommen und sofort mit 10000 Units Thrombin (Sigma) in 100 mM NaHCO₃, 500 mM NaCl, pH 8,3 gemischt.
   Die Lösung wurde 24 Stunden bei 4°C vorsichtig geschüttelt.
b) Blockierung:
   Das Gelmaterial wurde nach Absetzen mit 100 mM NaHCO₃, 500 mM NaCl, pH 8,3 gewaschen. Die Sepharose wurde dann mit 100 mM NaHCO₃, 500 mM NaCl, 1 M Ethanolamin pH 8,3 für 2 Stunden inkubiert.
c) Vorbereitung:
   Zur Entfernung des ungebundenen Thrombin wird das Gelmaterial vor Gebrauch noch einmal in der Säule mit 20 Säulenvolumen PBS pH 7,4 gewaschen.

### Beispiel 4

Bestimmung des Molekulargewichts durch Molekularsiebchromatographie.

Bei einer Flußgeschwindigkeit von 1 ml/min wurde durch Mono-Q®-Chromatographie gereinigtes Material in 20 mM Natriumphosphat, 150 mM NaCl, pH 7,5 auf einer Molekularsiebsäule des Typs TSK® (Pharmacia, Spherogel TSK 3000® SW, 7,5 mm Durchmesser, 60 cm Höhe) getrennt.

Ebenso wurde mit den Eichproteinen verfahren (Serumalbumin MW 67000 Da, Ovalbumin MW 45000 Da, Chymotrypsinogen A MW 25000 Da).

Der Logarithmus des Molekulargewichts der Eichproteine wurde gegen deren Elutionszeit in einem Diagramm aufgetragen.

In den fraktionierten Eluaten der Probe wurde die Thrombininhibition bestimmt.

Die Elutionszeit des Inhibitors ergab am Schnittpunkt mit der Eichgeraden den Logarithmus des gesuchten Molekulargewichts.

Durch diese Bestimmung wurde ein Molekulargewicht zwischen 22 000 und 28 000 Dalton bestimmt.

### Beispiel 5

Bestimmung des Molekulargewichts durch Tricine-SDS-Polyacrylamid Gel Elektrophorese.

(Literatur: Analytical Biochemistry, 166, 368 - 379 (1987) Tricine-Sodium Dodecyl Sulfate-Polyacrylamide Gel Electrophoresis for the Separation of Proteins in the range from 1 - 1000 kDa, Schägger, H. and von Jagow, G.)

Die Gelelektrophorese wurde entsprechend der Literaturvorschrift bei 20 mA und 1400 V, 30 Watt, durchgeführt.

Das nach dieser Methode bestimmte Molekulargewicht betrug 8000 ± 1500 Dalton.

Als Eichproteine dienten (Intaktes Myoglobin 17,2 kDa, Bromcyanpeptid Myoglobin I+II 14,6 kDa, Bromcyanpeptid Myoglobin I 8,2 kDa, Bromcyanpeptid Myoglobin II 6,4 kDa, Bromcyanpeptid Myoglobin III 2,6 kDa, Myoglobin 1-14)

### Beispiel 6

### Sequenzbestimmung des Inhibitors Reduktion und Carboxymethylierung.

2,8 ml Proteinlösung (0,029 mg/ml) wurden mit 0,28 ml Puffer (1 M Tris/HCl, 0,5 M Guanidinhydrochlorid, pH 8,6), gemischt. Danach wurden 0,116 ml Dithiothreitol (DTT, 10 mg/ ml) zugegeben und 10 Minuten bei 37°C inkubiert. Danach wurde nach Zugabe von 0,185 ml Jodacetamid (10 mg/ml) für 90 Minuten bei 37°C inkubiert. Die Reaktion wurde mit 0,073 ml DTT wie oben beendet.

Das Protein wurde durch erneute reversed phase HPLC an RP 318® gereinigt. Das Gemisch wurde auf eine Endkonzentration von 0,1 Gew.% Trifluoressigsäure (TFA) eingestellt und in einem HPLC System von Hewlett Packard (HP 1090 Liquid Chromatograph) getrennt. Mit Lösungsmittel A (0,1 % TFA, 100 % H₂O) wurde 5 Minuten gewaschen. Dann wurde der Anteil von Lösungsmittel B (90 % Acetonitril, 10 % H₂O, 0,1 Gew.% TFA) im Verlauf von 120 Minuten auf 50 % angehoben. Die Absorption des Eluats bei 214 und 280 nm wurde gemessen. Absorbierende Fraktionen wurden gesammelt. Das Protein wurde durch SDS-Gelelektrophorese identifiziert und einer Sequenzanalyse auf einem Applied Biosystems 477 A Protein Sequencer nach Vorschrift des Geräteherstellers unterworfen.

Folgende Sequenz wurde erhalten (SEQ ID NO:1):
Leu-Asn-Val-Leu-Cys-Asn-Asn-Pro-His-Thr-Ala-Asp-Cys-Asn-Asn-Asp-Ala-Gln-Val-Asp-Arg-Tyr-Phe-Arg-Glu-Gly-Thr-Cys-Leu-Met-Ser-Pro.

### Beispiel 7

Bestimmung des isoelektrischen Punktes durch isoelektrische Fokussierung

Die Bestimmung wurde mit einem LKB Multiphor 2117 (Horizontalsystem) und einem LKB powersupply 2103 durchgeführt. Es wurden Fertiggele eingesetzt (Pharmacia Ampholine PAGplate pH 3,5 - 9,5). Als Standarproteine wurden Amyloglucosidase pH 3,5; Soyabean Trypsin Inhibitor, pH 4,55; β-Lactoglobulin A, pH 5,2; Bovine carbonic anhydratase, pH 5,85; Human carbonic anhydratase, pH 6,55; Horse myoglobin, pH 6,85 und 7,35: Lentil Lectin, pH 8,15, 8,45, 8,65 und Trypsinogen, pH 9,3 eingesetzt.

Die Bedingungen der Fokussierung: 1500 Volt, 30 Watt.
- Puffer:: Anode 1M Phosphorsäure
Kathode 1M Natronlauge

Die Platten wurden 30 Minuten zur Ausbildung eines pH-Gradienten vorfokussiert. Die Proben wurden auf Filterplättchen aufgetragen, die auf dem Gel lagen. Für 30 Minuten wurde weiterfokussiert, die Filterplättchen abgenommen, und nach weiteren 30 Minuten wurde die Fokussierung beendet. Die Gele wurden sofort in 2 mm Scheiben geschnitten und in dest. Wasser überführt. Über Nacht eluierte das Protein aus den Gelscheiben. Durch einen Thrombininhibitionstest wurde die Lage des Thrombininhibitors bestimmt. Der pH-Wert kann auch direkt durch eine pH-Elektrode bestimmt werden. Hirudin, als Vergleichssubstanz hatte einen isoelektrischen Punkt von pH 3,5 und niedriger. Der neue Inhibitor hatte einen isoelektrischen Punkt von pH kleiner als 3,8.

### Beispiel 8

Herstellung einer DA-Sequenz, die für ein thrombininhibitorisches Protein codiert.
a) Isolierung von RNA und Herstellung einer cDNA-Bank
   Gesamt-RNA aus ganzen Tieren der Spezies Ornithodoros moubata wurde durch Aufschluß in Guanidiniumthiocyanat gewonnen. Dabei wurde mit Materialien und nach Anleigung des "RNA Isolation Kit" der Firma Stratagene, La Jolla, CA, USA (Katalog Nr: 200345) gearbeitet.
   Die polyadenylierte messenger RNA wurde aus der Gesamt-RNA durch oligo (dT)-Affinitätsseparation selektiert. Dieses Verfahren wurde mit Materialien und nach Anleitung des "PolyATtract mRNA Isolation System" der Firma Promega, Madison, WI, USA (Katalog Nr: Z5200) durchgeführt.
   Aus polyadenylierter messenger RNA wurde mit Materialien und nach Anleigung des "ZAP-cDNA Synthesis Kit" der Firma Stratagene, La Jolla, CA, USA (Katalog Nr: 200400) cDNA synthetisiert, die dann in die Eco RV Restriktionsstelle des Plasmids p Bluesecript II SK der Firma Stratagene, La Jclla, USA (Katalog Nr: 212205) kloniert wurde.
b) Herstellung von Oligonukleotidproben für die PCR
   Zur Klonierung von cDNA-Fragmenten mit Hilfe der Polymerase-Kettenreaktion (PCR, s. "Molecular Cloning" 2nd edition (1989), Sambrook, J. et al., CSH-Presse, Seite 14.1 ff.) wurde von Peptiden der in Beispiel 6 beschriebenen Protein-Sequenz ausgegangen.
   Unter Zugrundelegung des genetischen Codes läßt sich aus der Peptidsequenz I):
   NH₂-Cys-Asn-Asn-Pro-His-Thr-Ala (Pos 5-11)
   die Nukleinsäuresequenz (Seq ID NO:2):
   5'-TGY AAY AAY CCN CAY ACN GC-3'
   und aus der Peptidsequenz II):
   NH2-Pro-His-Thr-Ala-Asp-Cys-Asn (Pos 8-14)
   die Nukleinsäuresequenz (Seg ID NO: 3)
   5'-CCN CAY ACN GCN GAY TGY AA TG-3'
   des kodierenden DNA-Stranges herleiten. Wegen der bekannten Degeneration des genetischen Codes sind an manchen Positionen mehrere Nukleotide (N: A, C, G, T; Y: C, T;) einsetzbar. Damit ergibt sich für SEQ ID NO: 2 eines 256fache und für SEQ ID NO: 3, eine 512fache Gemischkomplexität. Die genannten Sequenzen wurden als Oligonukleotide synthetisiert.
   Die Synthesen wurden mit einem Applied Biosystems Typ 360A DNA-Synthesizer durchgeführt. Die Oligonukleotide wurden naoh Entfernung der Schutzgruppen gelelektrophoretisch über ein Polyacrylamid/Harnstoff-Gel gereinigt.
c) PCRs und Klonierung einer Teil-cDNA-Sequenz
   Die Polymerase-Kettenreaktion wurde nach bekannten Protokolle durchgeführt (s. "Molecular Cloning", 2nd edition (1989), Sambrook, J. et al., CSH-Presse, Seite 14.1 ff.). Dazu wurde ein "DNA Thermal Cycler" der Firma Perkin Elmer benutzt. Dabei wurde das Prinzip der "verschachtelten Primer" nach Frohmann, M.A. et al. (Proc. Natl. Acad. Sci. USA (1988) 85, 8998-9002) verwendet und nach Fritz, J.D. et al. (Nucl. Acids Res. (1991) 19, 3747) modifiziert angewendet.
   Im einzelnen wurde die cDNA aus a) mit jeweils 20 pmol der Oligonukleotide SEQ ID NO: 2 und dem synthetischen T7 Primer (abgeleitet aus p Bluescript II SK) ampliziert. Die Bedingungen waren dabei: 1'95°C; 3'72°C für 35 Zyklen.
   Die PCR-Produkte wurden elektrophoretisch auf einem 1,2%igen LMP-Agarose TBE-Gel aufgetrennt.
   Aus dem Gel wurden über die gesamte Länge des "Schmiers" 5 Gelscheibchen geschnitten und diese als separate Fraktionen mit DNA-Fragmenten steigender Molmasse geschmolzen.
   Aliquots dieser Fraktionen wurden dann separat in eine zweite PCR mit jeweils 20 pmol der Oligonukleotide ii) und dem synthetischen M13-20 Primer (abgeleitet aus p Bluescript II SK) eingesetzt.
   Dabei überschritt der Agaroseanteil nie 1/10 Volumen des PCR-Ansatzes. Reaktionsbedingungen: 1'95°C; 2'50°C; 3'72°C für 35 Zyklen.
   Die gelelektophoretische Auftrennung der Amplifikationsprodukte dieser Fraktionen ließ deutlich eine Reduzierung des komplexen Produktspektrums der ersten PCR in zu einer definierten Bande nach der zweiten PCR erkennen.
   Die solchermaßen selektrierten PCR-Produkte wurden nach Standardmethoden eluiert. Nach Subklonierung in die EcoRV-Schnittstelle des Vektors pBluescriptKS und Vermehrung des Plasmides in E.coli DH5alpha ergab die Sequenzanalyse eines Klones ein offenes Leseraster von 91 Aminosäuren (SEQ ID NO: 6), das mit der vorhergesagten Protein-Sequenz (SEQ ID:1) übereinstimmte.
   Die DNA und die daraus abgeleitete Aminosäuresequenz ist in SEQ ID NO: 6 dargestellt.
d) Heterologe Expression
   Zur heterologen Expression der 91 Aminosäuren langen Sequenz in E.coli wurden folgende Oligonukleotide synthetisiert:
   Nukleinsäuresequenz SEQ ID NO: 4
   und
   Nukleinsäuresequenz SEQ ID NO: 5

Dabei leitet sich SEQ ID NO: 4 aus der Seq ID NO: 1 her; es entspricht der Sequenz für die Aminosäuren von Position 1 bis 14; SEQ ID NO: 5 leitet sich aus der Seq ID NO: 6 ab und entspricht der Sequenz des Gegenstranges für die Aminosäuren 87 bis 91 sowie dem Stop Codon. Die zusätzlichen 15 Nukleotide am 5'Ende von SEQ ID NO: 5 sollen eine Sal I Schnittstelle zu Klonierungszwecken rekonstituieren.

Eine PCR mit diesen beiden Oligonukleotiden und der in c) klonierten DNA als "template" führte zur Isolation einer cDNA-Sequenz, die die vollständige codierende Region des o.a. Thrombininhibitors (Seg ID NO: 6) beinhaltet. Die Reaktionsbedingungen waren 10 µl "template", jeweils 20 pmol der Oligonukleotide SEQ ID NO: 4 und SEQ iD NO:5; 1'95°C; 2' 55°C; 2' 55°C; 3'72°C für 35 Zyklen.

Das PCR-Produkt wurde mit Sal I nachgeschnitten, gelelektrophoretisch analysiert und nach Standardmethoden eluiert.

Zur heterologen Expresion in E.coli wurde das PCR Fragment in den induzierbaren und im Periplasma exprimierenden Vektor pMal-p2 der Firma New England Biolabs, Beverly, MA, USA (Katalog Nr. 800-65 S) subkloniert.

Hierzu wurde pMa1-p2 mit Xmn I und Sal I verdaut, gelelektrophoretisch aufgetrennt und die Vektorbande nach Standardmethoden eluiert und mit dem PCR-Fragment ligiert.

Die Sequenz des neuen Thrombininhibitors (Seq ID NO: 6) wurde dadurch direkt an das 3'Ende des bakteriellen Gens für das Maltose-Binde-Protein (mal E) fusioniert.

Das Konstrukt wurde nach Standardvorschriften in den E.coli Stamm TB1 (New England Biolabs) transformiert.

Wachstum transformierter Zellen, Induktion mit IPTG und Aufarbeitung der Periplasmafraktion sowie Freisetzung des Thrombininhibitors durch Faktor Xa-Spaltung erfolgte genau nach den Angaben des Herstellers. Die einzelnen Schritte wurden durch SDS Gelelektrophorese analysiert.

Der rekombinant hergestellte Thrombininhibitor zeigte im Thrombinhemmtest eine Aktivität, die dem natürlichen Inhibitor aus Ornithodoros moubata voll vergleichbar war.

## Patentansprüche

1. Protein mit thrombininhibitorischer Wirkung aus Zecken der Gattung Ornithodoros, dadurch gekennzeichnet, daß es einen isoelektrischen Punkt von pH kleiner als 3,8, ein Molekulargewicht von 8000 ± 1500 Dalton, bestimmt durch SDS-Polyacrylamidgelelektrophorese, und die N-terminale Aminosäuresequenz Leu-Asn-Val-Leu-Cys-Asn-Asn-Pro-His-Thr-Ala-Asp-Cys-Asn-Asn-Asp-Ala-Gln-Val-Asp aufweist.

2. Protein nach Anspruch 1 mit der in SEQ ID NO: 7 dargestellten Aminosäuresequenz.

3. DNA-Sequenz, die für ein Protein gemäß Anspruch 1 codiert.

4. DNA-Sequenz nach Anspruch 3 mit der in SEQ ID NO: 6 dargestellten Nukleotidsequenz.

5. Expressionsvektor, der eine DNA-Sequenz gemäß Anspruch 3 enthält.

6. Verwendung eines Proteins gemäß Anspruch 1 zur Bekämpfung von Krankheiten.

## Claims

1. A protein with a thrombin-inhibitory action from ticks of the genus Ornithodoros, which has the following characteristics: it has an isoelectric point at a pH below 3.8, a molecular weight of 8000 ± 1500 Dalton, determined by SDS polyacrylamide gel electrophoresis, and the N-terminal amino-acid sequence Leu-Asn-Val-Leu-Cys-Asn-Asn-Pro-His-Thr-Ala-Asp-Cys-Asn-Asn-Asp-Ala-Gln-val-Asp.

2. A protein as claimed in claim 1 with the amino-acid sequence depicted in SEQ ID NO: 7.

3. A DNA sequence which codes for a protein as claimed in claim 1.

4. A DNA sequence as claimed in claim 3 with the nucleotide sequence depicted in SEQ ID NO: 6.

5. An expression vector which contains a DNA sequence as claimed in claim 3.

6. The use of a protein as claimed in claim 1 for controlling diseases.

## Revendications

1. Protéine à activité inhibitrice de la thrombine de tiques ou d'ixodes du genre Ornithodoros, caractérisée en ce qu'elle présente un point isoélectrique de pH inférieur à 3,8, un poids moléculaire de 8000 ± 1500 daltons, déterminé par électrophorèse sur gel de DSS-polyacrylamide et la séquence des aminoacides N-terminale Leu-Asn-Val-Leu-Cys-Asn-Asn-Pro-His-Thr-Ala-Asp-Cys-Asn-Asn-Asp-Ala-Gln-Val-Asp.

2. Protéine suivant la revendication 1 avec la séquence des aminoacides représentée dans SEQ ID NO: 7.

3. Séquence d'ADN qui code pour une protéine suivant la revendication 1.

4. Séquence d'ADN suivant la revendication 3 avec la séquences des nucléotides représentée dans SEQ ID NO: 6.

5. Vecteur d'expression qui contient une séquence d'ADN suivant la revendication 3.

6. Utilisation d'une protéine suivant la revendication 1 pour lutter contre des maladies.
